Europäisches Patentamt

European Patent Office

Office européen des brevets

(19)

(11) Publication number: 0 265 952
A2

# EUROPEAN PATENT APPLICATION

(21) Application number: 87115915.8

(22) Date of filing: 29.10.87

(51) Int. Cl.4: G01N 21/31 , A61B 5/00

(30) Priority: 29.10.86 JP 257669/86

(43) Date of publication of application:
04.05.88 Bulletin 88/18

(84) Designated Contracting States:
DE FR GB IT NL SE

(71) Applicant: Nihon Kohden Corporation
31-4, 1-chome, Nishiochiai
Shinjuku-ku Tokyo(JP)

(72) Inventor: Aoyagi, Takuo
Nihon Kohden Corporation 31-4, Nishiochiai
1-chome
Shinjuku-ku-ku Tokyo(JP)
Inventor: Sasaki, Tadashi
Nihon Kohden Corporation 31-4, Nishiochiai
1-chome
Shinjuku-ku-ku Tokyo(JP)
Inventor: Kobayashi, Nobutaka
Nihon Kohden Corporation 31-4, Nishiochiai
1-chome
Shinjuku-ku-ku Tokyo(JP)

(74) Representative: Patentanwälte Grünecker,
Kinkeldey, Stockmair & Partner
Maximilianstrasse 58
D-8000 München 22(DE)

(54) Apparatus for determining the concentration of a light-absorbing material in blood.

(57) An apparatus for very accurately determining the concentration of a light-absorbing material contained in blood flowing through a tissue which does not require the use of highly accurate components such as A/D converters having large numbers of bits. The apparatus operates according the principle that light beams of different wavelengths are absorbed by different amounts when passing through the tissue at different portions of the blood pulsation cycle. A digitization technique is employed whereby when limit values of the detected analog signal is reached, the input signal to an A/D converter is reset and digital output signal appropriately scaled.

FIG. 5

Xerox Copy Centre

# APPARATUS FOR DETERMINING THE CONCENTRATION OF A LIGHT-ABSORBING MATERIAL IN BLOOD.

## BACKGROUND OF THE INVENTION

The present invention relates to an apparatus for determining the concentration of a light-absorbing material in blood.

An oximeter is an example of an apparatus currently employed to determine the concentration of light-absorbing materials in blood. In a known type of oximeter, the oxygen saturation of blood in a living tissue sample is computed based on the fact that the relative quantities of two light beams having different wavelengths that pass through the tissue sample differ on account of blood pulsation. The operating principle of this type of oximeter is described hereinafter.

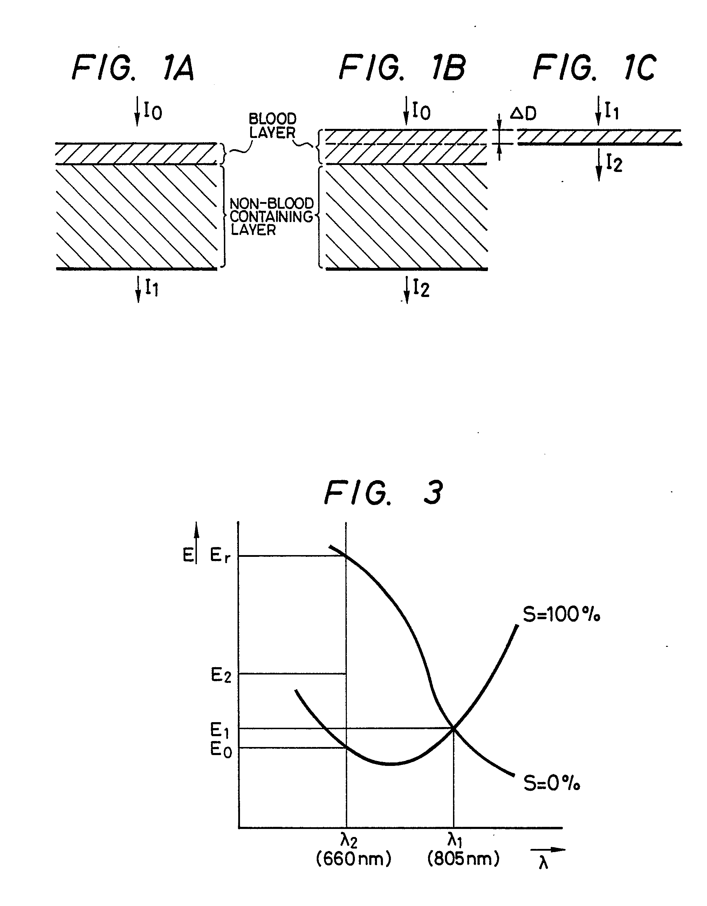

It is assumed first that a living tissue sample through which light is to pass is composed of a blood containing tissue layer and a non-blood tissue layer as shown in Fig. 1A. In this case, the light attenuation by the overall tissue sample is expressed by:

$$-\log (I_1/I_0) = A + B \quad ...(1)$$

where $I_0$: the quantity of incident light,

$I_1$: the quantity of transmitted light,

A : the amount of light attenuation by the non-blood containing tissue, and

B: the amount of light attenuation by the blood layer.

The light attenuation by the blood layer (B) is expressed by:

$$B = E \bullet C \bullet D \quad ...(2)$$

where E: the absorptivity coefficient of hemoglobin,

C: the concentration of hemoglobin in blood, and

D: the thickness of the blood containing layer.

Therefore, equation (1) can be rewritten as:

$$-\log (I_1/I_0) = -A + E \bullet C \bullet D \quad ...(3)$$

The thickness of the blood layer D varies due the normal arterial blood pulsation. It is assumed that the thickness of the blood layer changes by $\Delta D$ as shown in Fig. 1B. If the quantity of light transmitted through the blood layer changed in thickness by $\Delta D$ is written as $I_2$, analogy with equation (3) gives:

$$-\log (I_2/I_0) = A + E \bullet C \bullet (D + \Delta D) \quad ...(4)$$

Subtracting equation (4) from equation (3),

$$-\{\log (I_1/I_0) = \log (I_2/I_0) \} = -EC\Delta D, \text{ or}$$

$$-\log (I_2/I_1) = EC\Delta D \quad ...(5)$$

As can be seen from equation (3), equation (5) is equivalent to the expression of light attenuation for the case where incident light having an intensity of $I_1$ passes through a blood layer with a thickness of $\Delta D$ to produce light transmission in a quantity $I_2$. This relation is depicted in Fig. 1C.

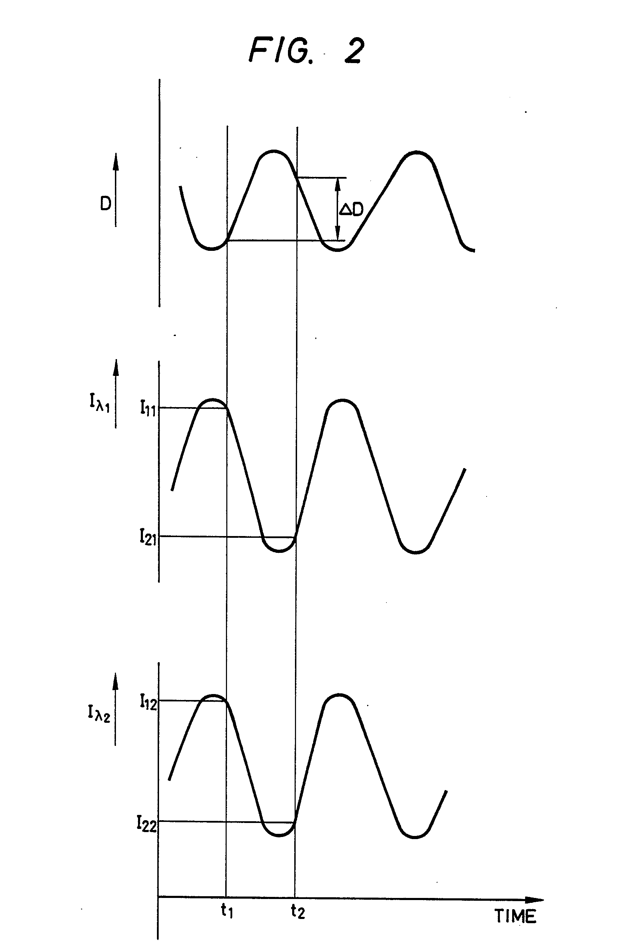

Next will be considered the case where two light beams having different wavelengths are transmitted through a blood containing layer at the measurement site. Fig. 2 shows the relationship between the thickness of the blood layer D and each of $I_1$ ( the quantity of transmitted light at a wavelength of $\lambda_1$ ) and $I_2$ (the quantity of transmitted light having a wavelength of $\lambda_2$). If the change in the thickness of the blood layer that occurs between two points in time $t_1$ and $t_2$ is written as $\Delta D$, and if the values of $I_1$ and $I_2$ at time $t_1$ are written as $I_{11}$ and $I_{12}$, respectively, with the values of $I_1$ and $I_2$ at time $t_2$ being written as $I_{21}$ and $I_{22}$, respectively, the following relations are established in consideration of equation (5):

For the first wavelength $\lambda_1$:

$$-\log (I_{21}/I_{11}) = E_1 C \Delta D \quad ...(6)$$

For the second wavelength $\lambda_2$:

$$-\log (I_{22}/I_{12}) = E_2 C \Delta D \quad ...(7)$$

where $E_1$ is the absorptivity coefficient of the blood for light at the wavelength $\lambda_1$ and $E_2$ is the absorptivity coefficient of the blood for light at the wavelength $\lambda_2$.

Equations (6) and (7) can be rewritten as follows:

$$\log (I_{11}/I_{21}) = E_1 C \Delta D \quad ...(8)$$

$$\log (I_{12}/I_{22}) = E_2 C \Delta D \quad ...(9)$$

Dividing equation (9) by equation (8) and writing the quotient as $\Phi$,

$$\Phi = \{\log (I_{12}/I_{22})\}/\{\log (I_{11}/I_{21})\} = E_1/E_2 \quad ...(10)$$

Since equation (10) does not contain the term $\Delta D$, the times $t_1$ and $t_2$ may be any two values.

Equation (10) can be rewritten as:

$$E_2 = \Phi \bullet E_1 \quad ...(11)$$

If the absorptivity coefficient $E_1$ in equation (11) is known, $E_2$ can be determined by calculating $\Phi$. As equation (10) shows, $\Phi$ can be determined by calculating $\log (I_{11}/I_{21})$ and $\log (I_{12}/I_{22})$, and, as already mentioned, $\log (I_{11}/I_{21})$ can be determined by measuring $I_{11}$ and $I_{21}$ (the quantities of transmitted light at the wavelength $\lambda_1$ at any two points in time), while $\log (I_{12}/I_{22})$ can be determined by measuring $I_{12}$ and $I_{22}$ (the quantities of transmitted light at the wavelength $\lambda_2$ at the aforementioned any two points in time).

Using $E_2$, the oxygen saturation S of the sampled blood may be calculated by the following procedures.

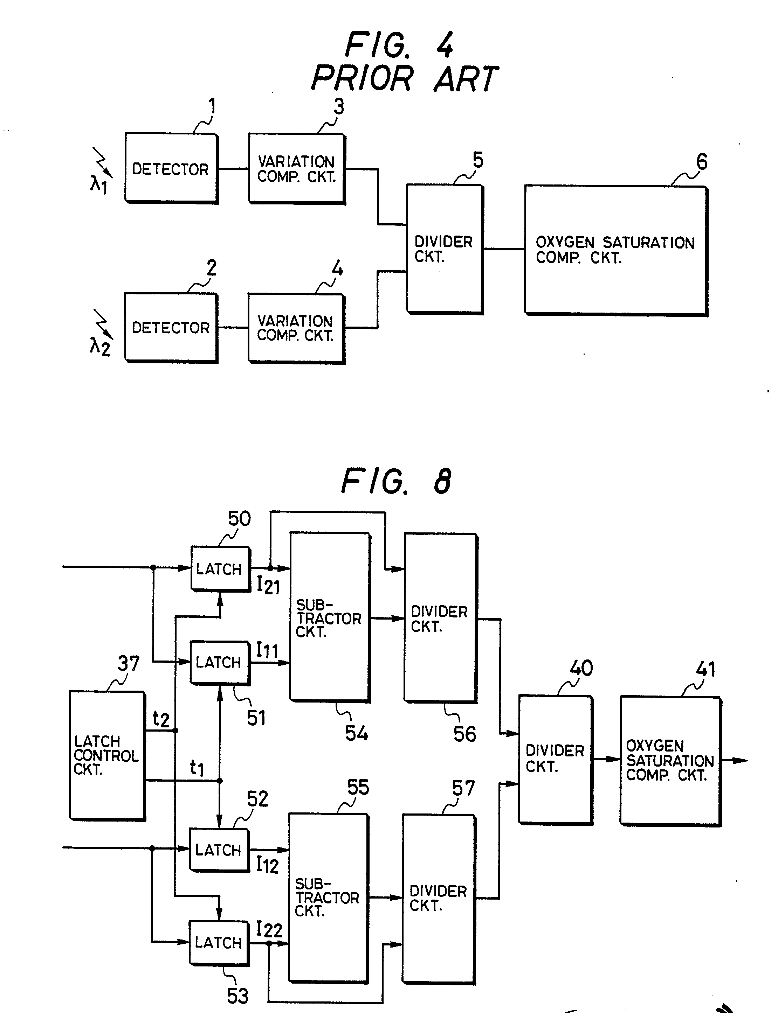

The absorptivity coefficient E of the blood versus the wavelength $\lambda$ of light with which a living body is irradiated is shown in Fig. 3 for S - 0% and S = 100%. The wavelength at which the curve for S = 0% crosses the curve for S = 100% is selected as the first wavelength $\lambda_1$, which falls at

805 nm in Fig. 3. The absorptivity coefficient $E_1$ for the light beam having the wavelength $\lambda_1$ is insensitive to changes in the oxygen saturation of blood S. Accordingly, a wavelength different from $\lambda_1$ is selected as the second wavelength $\lambda_2$, which falls, for instance, at 660 nm in Fig. 3. At the wavelength $\lambda_2$, the absorptivity coefficient assumes the value $E_r$ when S = 0% and the value $E_0$ if S = 100%. $E_2$ is a value between $E_0$ and $E_r$. Using $E_r$, $E_0$ and $E_2$, S can be calculated by the following equation:

$$S = (E_2 - E_r) / (E_0 - E_r) \quad ...(12)$$

An apparatus which determines the oxygen saturation S of blood using the procedure described above is shown schematically in Fig. 4. Detectors 1 and 2 receive light beams that have passed through a living tissue sample and which have wavelengths of $\lambda_1$ and $\lambda_2$, respectively, and produce output signals indicative of the intensities of the two beams. Variation computing circuits 3 and 4 compute the respective amounts of light attenuation on the basis of the changes in the detection signals produced by detectors 1 and 2 at two identical points in time. In other words, using $I_{11}$ and $I_{12}$ representing the quantities of transmitted light at time $t_1$, as well as $I_{21}$ and $I_{22}$ representing the quantities of transmitted light at time $t_2$ (see Fig. 2), the circuits 3 and 4 compute log ($I_{11}/I_{21}$) and log ($I_{12}/I_{22}$), respectively, which are the left side of equations (8) and (9). As a result, the variation in light attenuation due to the change in blood thickness ($\Delta D$) on the right side of each of equations (8) and (9) is determined. Using the calculation results produced by the circuits 3 and 4, a divider circuit 5 determines $\phi$ expressed by equation (10). In the next step, an oxygen saturation computing circuit 6 computes S from equations (11) and (12) using the value of $\phi$ calculated by the divider circuit 5 and the preliminarily stored values of $E_1$, $E_r$ and $E_0$ as indicated in Fig. 3.

The apparatus of the type described above has conventionally employed analog computing circuitry, but with recent advances in microcomputer technology, the use of a digital computing circuitry in medical systems has gained prevalence. Data processing with digital computing circuitry of course has the advantage of high-speed calculation of correct measurements.

In spite of this great advantage, digital processing has problems. For the accuracy of such measurements, the A/D converter employed for digitizing the output signals from the light detectors is required to have a high resolution.

The quantity of transmitted light may be regarded as being composed of AC component which varies in response to blood pulsation and a DC component which is independent of blood pulsation. The AC component is less than 10% of the DC component. The DC component is still variable with other factors. The accuracy of A/D conversion of both AC component and DC component must be satisfactory. Twelve-bit A/D converters, as are readily available on the market, are unable to achieve A/D conversion of the AC component with satisfactory accuracy.

## SUMMARY OF THE INVENTION

The present invention has been accomplished in order to solve these problems of the prior art. An object, therefore, of the present invention is to provide an apparatus for determining the concentration of a light-absorbing material in blood that ensures the correct determination of the concentration of the light-absorbing material while using an A/D converter of low resolution.

The stated object of the present invention is attained by an apparatus for determining the concentration of a light-absorbing material in blood that comprises: a detector for detecting the intensities of light beams having different wavelengths which have passed through a living tissue; a subtractor circuit which receives the detection signal from said detector as one input signal thereof; an A/D converter for digitizing the output signal from said subtractor circuit; a D/A converter whose output signal serves as the other input signal for said subtractor circuit; means for being controlled by the output signal from said A/D converter and supplying said output signal to said D/A converter; weighting addition means for summing the output signal from said A/D converter and the output signal from said controlling means with the respective output signals being weighted, and concentration computing means which computes the concentration of a light-absorbing material of interest on the basis of the output signal produced by said weighting addition means for each wavelength of light.

In the apparatus of the present invention having the arrangement described above, the magnitude of the value of the signal to be supplied to the D/A converter is determined in accordance with the magnitude of the value of the output signal from the A/D converter. The advantage of this design is that, if the value of the detection signal from the detector exceeds the permissible level for the A/D converter, the subtractor circuit will supply the A/D converter with a signal whose magnitude is equal to the value of the detection signal minus the value of the output signal from the D/A converter, and hence the LSB of the A/D converter may be weighted by a small value. The weighting addition means sums the output signal from the A/D converter and the input signal to the D/A converter after appropriately weighting the values of the respective signals. The weighting addition means then outputs to the concentration computing means a signal whose value is dependent on the output signal from the detector. The concentration computing means computes the concentration of the light-absorbing material of interest on the basis of the results of the outputted by the weighting addition means.

## BRIEF DESCRIPTION OF THE DRAWINGS

Fig. 1A to 1C illustrate schematically a cross section of a tissue containing blood vessels;

Fig. 2 is a waveform diagram showing changes in the thickness of the tissue of Figs. 1A to 1C and intensities of two different light beams passing through the tissue;

Fig. 3 is a graph showing the relationship between absorptivity and wavelength;

Fig. 4 is a block diagram of an apparatus constructed on the basis of the principles illustrated in Figs. 1A to 3;

Fig. 5 is a schematic block diagram of an apparatus constructed according to a first preferred embodiment of the present invention;

Fig. 6 shows graphically the operation of the apparatus shown in Fig. 5; and

Figs. 7 and 8 illustrate an apparatus according to another embodiment of the present invention;

## DESCRIPTION OF THE PREFERRED EMBODIMENTS

Preferred embodiments of the present invention are hereunder described with reference to the accompanying drawings.

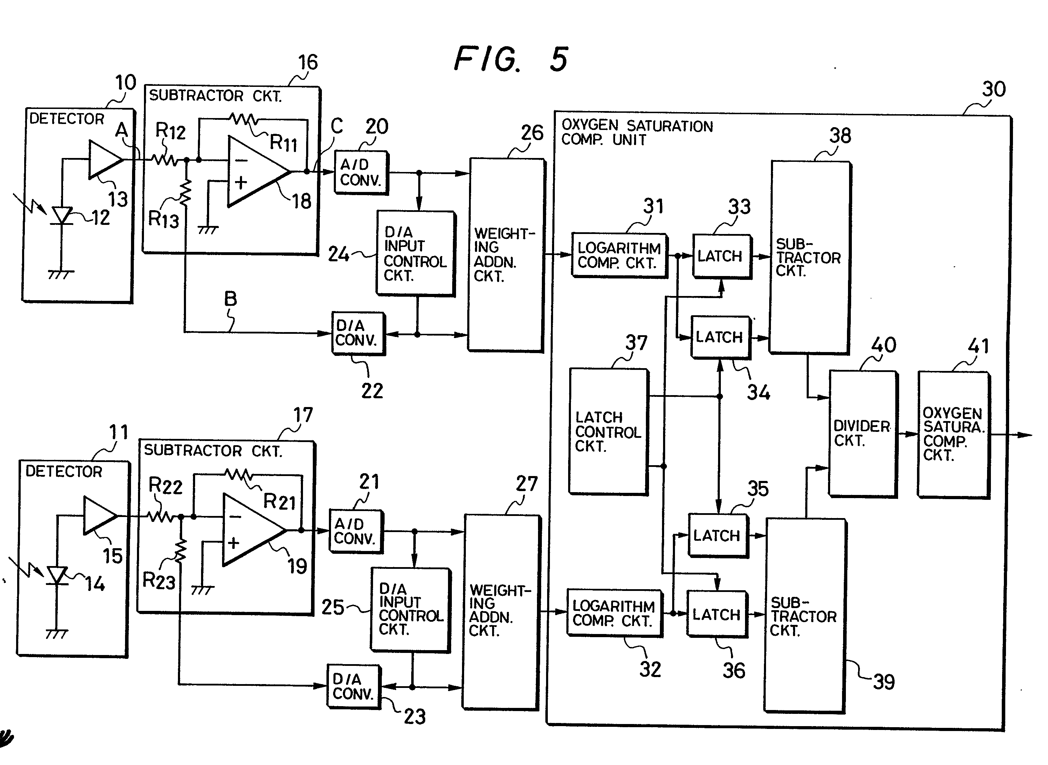

Fig. 5 is a schematic block diagram of an oximeter constructed according to a preferred embodiment of the present invention. Shown at 10 and 11 in Figs. 5 are detectors which detect the intensities of radiation of light beams having wavelengths of $\lambda_1$ and $\lambda_2$ that have passed through a living tissue. The detector 10 is composed of a photodiode 12 and an amplifier 13, and the detector 11 is composed of a photodiode 14 and an amplifier 15. The output signals from the detectors 10 and 11 are supplied to subtractor circuits 16 and 17, respectively, at first input terminals thereof. The subtractor circuit 16 is composed of an operational amplifier 18 and resistors $R_{11}$, $R_{12}$ and $R_{13}$, and the subtractor circuit 17 is composed of an operational amplifier 19 and resistors $R_{21}$, $R_{22}$ and $R_{23}$.

Shown at 20 and 21 are A/D converters which perform A/D conversion of the output signals from the subtractor circuits 16 and 17, respectively. Indicated at 22 and 23 are D/A converters. The output of the D/A converter 22 (23) is connected to the other input terminal of the subtractor circuits 16 (17). Shown at 24 and 25 are D/A input control circuits, each of which is composed of a logic circuit. Each time the value of the output signal from the A/D converter 20 (21) reaches a predetermined upper limit, the D/A input control circuit 24 (25) adds "1" to the digital value of the signal then being supplied to the D/A converter 22 (23) (this adding operation is hereafter referred to as "counting up") and maintains that state. Each time the value of the output signal from the A/D converter 20 (21) reaches a predetermined lower limit, the D/A input control circuit 24 (25) subtracts "1" from the digital value of the signal then being supplied to the D/A converter 22 (23) (this subtracting operation is referred to hereafter as "counting down") and maintains that state.

Shown at 26 and 27 are weighting addition circuits. The circuit 26 weights the value of the output signal from the A/D converter 20 and the value of the input signal to the D/A converter 22 in accordance with the LSB of each converter, and sums the weighted values. In a like manner, the circuit 27 weights the value of the output signal from the A/D converter 21 and the value of the input signal to the D/A converter 23 in accordance with the LSB of each converter, and sums the weighted values.

Shown at 30 is oxygen saturation computing unit, which is composed of logarithm computing circuits 31 and 32, latches 33, 34, 35 and 36, a latch control circuit 37, subtractor circuits 38 and 39, divider circuit 40 and an oxygen saturation computing circuit 41. The logarithm computing circuits 31 and 32 calculate the logarithms of the output signal values from the weighting addition circuits 26 and 27, respectively. A pair of latches 33 and 34 (35 and 36) latch the output signal from the logarithm computing circuit 31 (32). The operational timing of these latches is controlled by the latch control circuit 37. Subtractor circuit 38 calculates the difference between the values of the signals held in the latches 33 and 34, and the subtractor circuit 39 calculates the difference between the values of the signals latched in the latches 35 and 36. The divider circuit 40 calculates the ratio of the results of calculation performed by subtractors 38 and 39. The oxygen saturation computing circuit 41 computes the oxygen saturation of the blood in the tissue on the basis of the calculation results provided by the divider circuit 40.

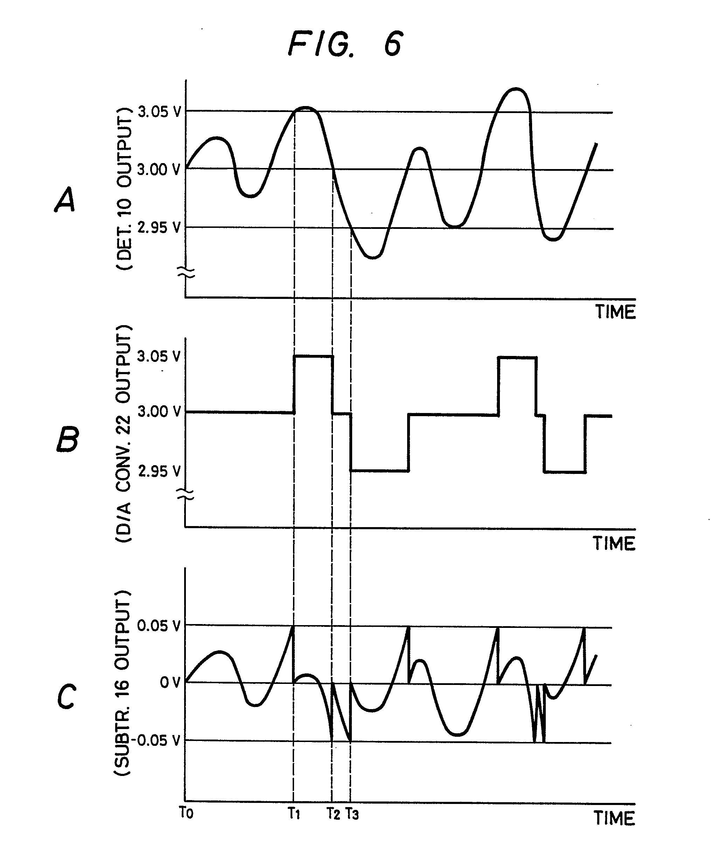

The apparatus having the composition described above operates in the following manner, the description of which assumes that the detector 10 converts incoming light with a wavelength of $\lambda_1$ to an electrical signal A as shown in Fig. 6. Signals B and C shown in Fig. 6 are the output signals from the D/A converter 22 and subtractor circuit 16 respectively, which vary with changes in the signal A. The relationship between these three signals is described hereinafter.

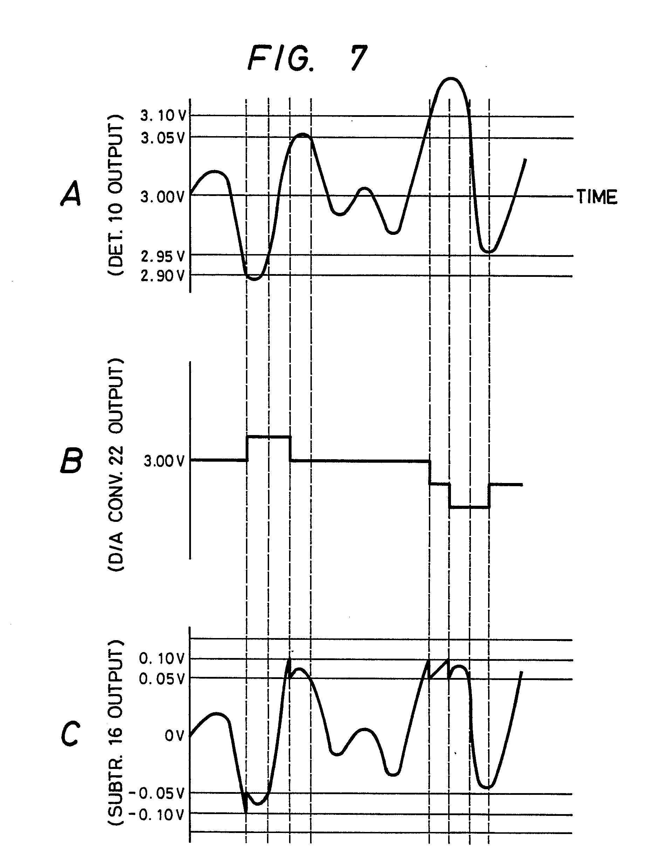

It is first assumed that both the signals A and B have a voltage of 3.00 volts at time $T_0$, while the signal C has a voltage of zero volts. When the voltage of the signal A increases to 3.05 volts at time $T_1$, the signal C reaches 0.05 volts which is the upper limit for the A/D converters. As a result, the D/A input control circuit 24 counts up the value of the input signal to D/A converter 22 by "1". Since the LSB of the D/A converter 22 is weighted by 0.05 volts, the voltage of the signal B is increased to 3.05 volts. Therefore, the signal C is reduced to zero volts. If the voltage of the signal A is 3.00 volts at time $T_2$, the signal C drops to -0.05 volts, which is the lower limit for the A/D converters. As a result, the D/A input control circuit 24 counts down the value of input signal to the D/A converter 22 by "1", causing the signal B to drop to 3.00 volts while increasing signal C to zero volts. If the voltage of the signal A is 2.95 volts at time $T_3$, the signal C is also at -0.05 volts. Therefore, the D/A input control circuit 24 counts down the value of input signal to the D/A converter 22 by "1", causing the signal B to drop to 2.95 volts, while increasing the signal C to zero volts. By repeating the same procedures, the fluctuations of the voltage of signal C that occur as a result of the signal A exceeding 3.05 volts falling below 2.95 volts can be maintained between the limits of -0.05 volts and +0.05 volts, as shown in Fig. 7. The weighting adder circuit 26 then weights the digital values of the signals C and B and sums the thus-weighted values. Therefore, the output signal from the weighting adder circuit 26 correctly indicates the digital value of the signal A from the detector 10.

If, in the embodiment shown above, the LSB of the A/D converter 20 is weighted by 0.01 volts, the converter does not need a resolution of more than four bits since the difference between -0.05 volts and +0.05 volts is 0.1 volts. On the other hand, if the signal A is to be directly subjected to A/D conversion (namely, the range of voltages that have to be accommodated is from 0 to 3.05 volts and upward), the A/D converter is required to have a resolution of 9 bits, assuming that its LSB is also weighted by 0.01 volts. In order to allow for the use of a four-bit A/D converter, its LSB must be weighted by approximately 0.2 bolts. It is therefore apparent that the apparatus of the present invention has the advantage of yielding accurate digital values using an A/D converter of low resolution (i.e., a reduced number of bits).

When light having a wavelength of $\lambda_2$ is received by the detector 11, it is converted to an electrical signal and subsequently processed to be outputted from the weighting adder circuit 27 through steps which are identical to those described above.

The oxygen saturation computing unit 30 computes the oxygen saturation of the blood on the basis of the signals supplied from the weighting adder circuits 26 and 27. The operation of the computing unit 30 is described hereinafter.

For the purposes of the following discussion, it is assumed that the weighting adder circuits 26 and 27 supply the logarithm computing circuits 31 and 32 with values that are dependent on the received intensities of light beams having different wavelengths $\lambda_1$ and $\lambda_2$ as shown in Fig. 2. The logarithm computing circuits 31 and 32 compute the logarithms of the supplied signal values. Th latch control circuit 37 outputs a timing signal to each of the latches 34 and 35 at time $t_1$ (see Fig. 2), whereupon the values $I_{11}$ and log $I_{12}$ are latched in latches 34 and 35, respectively. In the next step, the latch control circuit 37 outputs a timing signals to each of the latches 33 and 36 at time $t_2$ (see Fig. 2), whereupon the values of log $I_{21}$ and log $I_{22}$ are latched in latches 33 and 36, respectively. Subtractor circuit 38 then calculates (log $I_{11}$ -log $I_{21}$) using the value log $I_{11}$ stored in the latch 34 and the value log $I_{21}$ stored in the latch 33. In other words, the subtractor circuit 38 produces log $(I_{11}/I_{21})$. In alike manner, the subtractor circuit 39 determines and produces log $(I_{12}/I_{22})$. Using the computation results from the subtractor circuits 38 and 39, the divider circuit 40 calculates log $(I_{12}/I_{22})$/log $(I_{11}/I_{21})$ to determine $\Phi$ in equation (10). The oxygen saturation computing circuit 41 holds the values of $E_1$, $E_r$ and $E_0$ shown in Fig. 3 and computes the oxygen saturation S in accordance with equations (11) and (12) using these values and the value of $\Phi$ provided by the divider circuit 40. The value of S so computed is displayed on a display unit (not shown).

The D/A input control circuits 24 and 25 perform the following operations.

The D/A input control circuit 24 operates by referencing the values of backup lines and return lines as noted in the graph showing signal C in Fig. 7. The signal C shown in Fig. 7 is the same as signal C in Fig. 6 in that both are output signals from the subtractor circuit 16, and the signals A and B shown in Fig. 7 are output signals form the detector 10 and the D/A converter 22, respectively. In the embodiment being discussed, the D/A input control circuit 24 controls the output signal from the D/A converter 22 in such a way that the signal C, when it reaches the value indicated by either of the two backup lines, will be returned to the value of the associated return line. The end lines shown in association with the signal C in Fig. 7 indicate the values of the upper and lower limits for the A/D converter 20 to be employed in practice. In accordance with this embodiment, the input signal C to the A/D converter 20 (the output signal from the subtractor circuit 16) is allowed to fluctuate between the two backup lines, thereby reducing the resolution (number of bits) needed for the A/D converter 22. The same modification can be made to the other D/A input control circuit 25.

In the embodiments described above, first and second detectors 11 and 12 are used to detect respective two beams of light having different wavelengths, and the output signals from these detectors are processed by two associated subtractor circuits 16 and 17, A/D converters 20 and 21, D/A converters 22 and 23, D/A input control circuits 24 and 25, and two weighting adder circuits 26 and 27. In other words, signal processing is carried out in two channels in the embodiments described above. However, if operations are performed on a time-sharing basis in such a way that two beams of light having different wavelengths are received alternately rather than simultaneously, with the other circuits operating in synchronism with the alternate reception of the beams of different wavelengths, the apparatus of the present invention can be operated using only a signal channel, namely, the elements from the detector to the weighting adder circuit.

Fig. 8 shows another arrangement that can be used for the oxygen saturation computing unit 30 shown in Fig. 5. In Fig. 8, components that are identical to those shown in Fig. 5 are identified by like reference numerals.

If $\Phi$ in equation (10), namely, $\log (I_{12}/I_{22})/\log (I_{22}/I_{21})$ is determined in the divider circuit 40, the oxygen saturation of the blood can be determined by the oxygen saturation computing circuit 41. As already mentioned, $\Phi$ can be determined from $\log (I_{12}/I_{22})$ and $\log (I_{11}/I_{21})$. The term $\log (I_{12}/I_{22})$ can be rewritten as $\log (I_{12}/I_{22}) = \log \{1 + (I_{12} - I_{22})/I_{22}\}$. Since $I_{12} - I_{22} << I_{22}$, the following approximation is valid:

$$\log (I_{12}/I_{22}) = (I_{12} - I_{22})/I_{22} \quad ...(13)$$

In a like manner, the following approximation is valid:

$$\log (I_{11}/I_{21}) = (I_{12} - I_{22})/I_{21} \quad ...(14)$$

Therefore, instead of determining $\log (I_{12}/I_{22})$ and $\log (I_{11}/I_{21})$, $(I_{12} - I_{22})/I_{22}$ and $(I_{11} - I_{21})/I_{21}$ may be used to obtain $\Phi$. The circuit shown in Fig. 8 is designed to operate on this principle. The operation of this circuit is as follows.

Latches 50 and 51 are supplied with the output signal from the weighting adder circuit 26 shown in Fig. 5, and latches 52 and 53 are supplied in a like manner with the output signal from the weighting adder circuit 27. These output signals take values that are dependent on the received intensities of light beams having wavelengths $\lambda_1$ and $\lambda_2$ which vary in magnitude as shown in Fig. 2. At time $t_1$ (see Fig. 2), the latch control circuit 37 outputs a timing signal to each of the latches 51 and 52, whereupon values representing the intensities of the transmitted light beams, namely, $I_{11} (\lambda_1)$ and $I_{12} (\lambda_2)$, respectively, are latched in latches 51 and 52, respectively. Then, at time $t_2$, the latch control circuit 37 outputs a timing signal to each of the latches 50 and 53, whereupon values representing the intensities of the transmitted light beams, namely, $I_{21} (\lambda_1)$ and $I_{22} (\lambda_2)$, are latched in the latches 50 and 53, respectively. The subtractor circuit 54 determines the difference between $I_{11}$ (latched in 51) and $I_{21}$ (latched in 50).

The subtractor circuit 55 determines the difference between $I_{12}$ (latched in 52) and $I_{22}$ (latched in 53). The divider circuit 56 then determines $(I_{11} - I_{21})/I_{21}$ using $(I_{11} - I_{21})$ as provided by the subtractor circuit 54 and $I_{21}$ held in the latch 50. In a like manner, the divider circuit 57 determines $(I_{12} - I_{22})/I_{22}$ using $(I_{12} - I_{22})$ determined by the subtractor circuit 55 and $I_{22}$ held in latch 53. The divider circuit 40 determines $\Phi$ from $(I_{11} - I_{21})/I_{21}$ and $(I_{12} - I_{22})/I_{22}$, which are the results of the calculations performed by the divider circuits 56 and 67, respectively. As in the embodiment shown in Fig. 5, the oxygen saturation computing circuit 41 calculates the oxygen saturation of the blood sample using $\Phi$ determined by the divider circuit 40. The circuit described above with reference to Fig. 8 has the advantage that $\Phi$ can be computed without performing logarithmic calculations.

In the embodiments described above, high-speed signal processing can be realized by using a microcomputer to operate the individual circuits dedicated to the processing of digital signals.

In accordance with the apparatus of the present invention for determining the concentration of a light-absorbing material in blood in response to changes in the quantity of light passing through part of a living tissue, the detection signal from a detector sensing the quantity of transmitted light is converted to a digital signal with reduced quantizing error using an A/D converter of lower resolution (reduced number of bits).

## Claims

1. An apparatus for determining the concentration of a light-absorbing material in blood based on a differential amount of transmission of light of different wavelengths passing through a living tissue due to blood pulsation, said apparatus comprising:

at least one light detecting and digitizing circuit comprising: a detector for detecting the intensity of said light passing through said tissue; a subtractor circuit receiving an output detection signal from said detector as one input thereof; an A/D converter for digitizing an output signal from said subtractor circuit, a D/A converter whose output signal is connected as the other input signal to said subtractor circuit, control means for generating an output signal to said D/A converter in response to an output signal from said A/D converter; and weighting addition means for summing said output signal from said A/D converter and said output signal from said control means with the respective output signals being weighted; and

concentration computing means for computing a concentration of a light-absorbing material in blood in said tissue in response to an output signal produced by said weighting addition means for each wavelength of light.

2. The apparatus for determining the concentration of a light-absorbing material in blood as recited in claim 1, wherein said weighting addition means weights said output signal from said A/D converter and said output signal from said control means in accordance with an LSB of A/D converter and an LSB of said output signal from said D/A converter.

3. The apparatus for determining the concentration of a light-absorbing material in blood as recited in claim 1, wherein there are provided two said light detecting and/or digitizing circuits, one for each of the light beams of different wavelengths passing through said tissue.

4. The apparatus for determining the concentration of a light-absorbing material in blood as recited in claim 1, wherein said control means generates said output signal to said D/A converter by adding a predetermined value to a digital value of the output signal then being supplied to said D/A converter when said output signal form said A/D converter reaches a predetermined upper limit and subtracts the predetermined value from said output signal then being supplied to said D/A converter when said output signal from said A/D converter falls below a predetermined lower limit.

5. The apparatus for determining the concentration of a light-absorbing material in blood as recited in claim 1, wherein said concentration computing means comprises: a first logarithm computing circuit receiving an output of a first said weighting addition means; first and second latches both receiving an output from said first logarithm computing means; a first subtractor circuit receiving as inputs outputs of said first and second latches; a second logarithm computing circuit receiving an output of a second said weighting addition means; third and fourth latches both receiving an output from said second logarithm computing means; a second subtractor circuit receiving as inputs outputs of said third and fourth latches; a latch control circuit for operation said first through fourth latches to store said output of said weighting addition means at respective times; a divider circuit receiving as inputs outputs of said first and second subtractor circuits; and an oxygen saturation computing circuit receiving as an input an output of said divider circuit.

6. The apparatus for determining the concentration of a light-absorbing material in blood as recited in claim 1, wherein said concentration computing means comprises; first and second latches both receiving an output from a first of said weighting addition means; a first subtractor circuit receiving as inputs outputs of said first and second latches; a first divider circuit receiving as inputs an output of said first subtractor circuit and said output of said first of said weighting addition means; third and fourth latches both receiving an output from a second of said weighting addition means; a second subtractor circuit receiving as inputs outputs of said third and fourth latches; a second divider circuit receiving as inputs an output of said second subtractor circuit and said output of said second of said weighting addition means; a latch control circuit for operating said first through fourth latches to store said output of said weighting addition means at respective times; a third divider circuit receiving as inputs outputs of said first and second divider circuits; and a concentration computing circuit receiving as an input an output of said third divider circuit.

7. The apparatus for determining the concentration of a light-absorbing material in blood as recited in claim 1, wherein an amplifying rate of an amplifier of said detector and/or amplifying rate of an amplifier of said subtractor circuit are adjustable in accordance with the output signal of said A/D converter.

8. The apparatus for determining the concentration of a light-absorbing material in blood as recited in claim 1, wherein each of the components of the apparatus is operated in a time-divisional manner, whereby each of the signal processing circuits is adapted to process a plurality of wavelength signals.

Neu eingereicht / Newly filed
Nouvellement déposé

## FIG. 1A    FIG. 1B    FIG. 1C

BLOOD LAYER

NON-BLOOD CONTAINING LAYER

## FIG. 3

$E_r$

$E_2$

$E_1$
$E_0$

S=100%

S=0%

$\lambda_2$
(660 nm)

$\lambda_1$
(805 nm)

$\lambda$

BAD ORIGINAL

Neu eingereicht / Newly filed
Nouvellement déposé

# FIG. 2

BAD ORIGINAL

Nachträglich / Neu eingereicht
Nouvellement déposé

# FIG. 4
## PRIOR ART

# FIG. 8

BAD ORIGINAL

# FIG. 5

BAD ORIGINAL

0 265 952

# FIG. 6

A  (DET. 10 OUTPUT)

3.05 V
3.00 V
2.95 V

TIME

B  (D/A CONV. 22 OUTPUT)

3.05 V
3.00 V
2.95 V

TIME

C  (SUBTR. 16 OUTPUT)

0.05 V
0 V
-0.05 V

T0    T1    T2 T3    TIME

BAD ORIGINAL

## FIG. 7

A (DET. 10 OUTPUT)
3.10 V
3.05 V
3.00 V — TIME
2.95 V
2.90 V

B (D/A CONV. 22 OUTPUT)
3.00 V

C (SUBTR. 16 OUTPUT)
0.10 V
0.05 V
0 V
-0.05 V
-0.10 V

BAD ORIGINAL